(19) Europäisches Patentamt / European Patent Office / Office européen des brevets

(11) **EP 3 892 262 A1**

(12) **EUROPEAN PATENT APPLICATION**

(43) Date of publication:
**13.10.2021 Bulletin 2021/41**

(51) Int Cl.:
***A61K 9/16*** *(2006.01)*       ***A61K 9/50*** *(2006.01)*

(21) Application number: **20168193.9**

(22) Date of filing: **06.04.2020**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Chemische Fabrik Budenheim KG
55253 Budenheim (DE)**

(72) Inventors:
• **Zakowiecki, Daniel
55257 Budenheim (DE)**
• **Heß, Tobias
65527 Niedernhausen (DE)**
• **Papaioannou, Markos
55291 Saulheim (DE)**

(74) Representative: **WSL Patentanwälte Partnerschaft
mbB
Kaiser-Friedrich-Ring 98
65185 Wiesbaden (DE)**

(54) **BEADS COMPRISING A BUFFER SYSTEM**

(57)     The present invention concern beads comprising a buffer system, pharmaceutically active pellets comprising the inventive beads, a method for producing beads comprising a buffer system and the use of a buffer system in beads, which are coated with a pharmaceutically active agent.

EP 3 892 262 A1

**Description**

The present invention is directed to beads for use in producing pharmaceutically active pellets being prepared by depositing a pharmaceutically active agent on the surface of said beads, wherein the beads comprise a pharmaceutically acceptable carrier material and a buffer system. Further, the invention is directed to pharmaceutically active pellets being prepared by depositing a pharmaceutically active agent on the surface of said beads, a method for producing said beads and the use of a buffer system in beads coated with a pharmaceutically active agent.

An increasing number of medicinal products are being marketed as multi-unit dosage forms. Multi-unit dosage forms are considered to be advantageous over single-unit dosage forms thanks to improved effectiveness and safety. These forms have been gaining popularity in recent years because of several reasons including the possibility of preparation of modified release formulations, physical separation of incompatible substances as well as overcoming the swallowing difficulties, especially in pediatric and elderly patients.

Multi-unit dosage forms are frequently produced by the layering technique which consists in applying layers of a pharmaceutically active agent (e.g. a drug solution, a drug suspension or a drug powder) onto beads (pellets), especially spherical beads, with dimensions lower than 1.5 mm. The beads consist of pharmaceutically acceptable inert compounds used as excipients in pharmaceutical technology such as microcrystalline cellulose (MCC), lactose, starch, sugars, silicon dioxide, carnauba wax. After loading with the pharmaceutically active agent, the drug-loaded beads are usually coated with one or more layers of polymeric materials.

EP 2 223 702 A1 discloses core particles for pharmaceutical preparations comprising a pharmaceutically acceptable inorganic material selected from calcium hydrogen phosphate, silicon dioxide, aluminum hydroxide and aluminum silicate. According to the examples, the core particles are produced by wet granulation and contain 100% of an inorganic material selected from the above-mentioned materials. In the wet granulation process disclosed, 20% of ethanol are used in order to improve the mechanical strength of the product. Accordingly, some of the ethanol used in the process can remain in the final product, which is undesirable in some specific applications.

EP 2 659 882 A1 discloses disintegrating particles. However, coating of disintegrating pellets with aqueous suspensions or solutions can prove to be challenging as even a small amount of liquid can start a disintegration process at least at the surface of the pellets.

EP 2 496 261 discloses a co-processed excipient made of a mixture of microcrystalline cellulose and calcium phosphate. The components are mixed in a weight ratio of from about 85:15 to about 55:45 microcrystalline cellulose to calcium phosphate. The co-processed product is intended to be used in processes involving multiple compaction steps. However, microcrystalline cellulose contains a quite high amount of water, which can be problematic in case of water-sensitive pharmaceutically active agents. Moreover, microcrystalline cellulose starts to swell upon contact with water. Therefore, in some applications the presence of large amounts of microcrystalline cellulose in pellets is undesirable.

However, the above-mentioned systems are not able to resist any changes in the local pH. Still, such pH changes can influence the dissolution behaviour, the dose properties and the stability of the pharmaceutical agent deposited on the beads. In addition, some pharmaceutically active agents such as some proton-pump inhibitors necessitate certain pH values to secure their chemical stability.

Therefore, there is a high need to provide beads, which have a higher resistance against changes in the local pH-value and/or ensure a constant pH-value throughout the pharmaceutically active pellet during the gastro-intestinal passage in order to improve dissolution behaviour and bioavailability of the pharmaceutically active agent. In addition, there is a need for beads providing a pH environment adjusted to the properties of the particular pharmaceutically active agent, which is coated on the beads.

This problem is solved by providing beads for use in producing pharmaceutically active pellets being prepared by depositing a pharmaceutically active agent on the surface of said beads, wherein the beads comprise

a) a pharmaceutically acceptable carrier material and
b) a buffer system,
wherein
c) the amount of the buffer system is at least 0.1 wt.-% in relation to the total weight of the beads and
d) the beads comprise less than 0.5 wt.-% active pharmaceutical agent in relation to the total weight of the beads.

**Feature a)**

The inventive beads comprise a pharmaceutically acceptable carrier material, wherein the term "pharmaceutically acceptable material" as used herein, refers to materials that are generally regarded as safe (GRAS classification of the American Food and Drug Administration, USFDA) for use in pharmaceutical products, particularly when the compositions are to be administered by the oral route.

In a preferred embodiment of the invention, the content of volatile components (e.g. water) of the beads is very low. Such beads are suitable for processing water-sensitive pharmaceutically active agents. In some embodiments of the invention, the content of volatile components is less than 2 wt.-% or even less than 1 wt.-%. The content of volatile components is expressed as loss on drying (LoD) and is determined by thermogravimetric analysis. In the thermogravimetric analysis, the spherical beads are dried using a sensitive electrobalance at a temperature of 105 °C to constant mass, which means a difference in weight per one minute of less than 0.05 wt.-%, preferably less than 0.02 wt.-%.

The moisture content is defined as the percentage loss in weight of the sample during the thermogravimetric analysis.

**Feature b)**

The most important feature of the inventive beads is their ability to maintain a relatively constant pH value in response to the appearance of small amounts of acids or bases in the internal or external environment. This effect is achieved by the buffer system of the present invention. A buffer system is a combination of substances whose pH value changes much less when an acid or a base is added than would be the case in an unbuffered system. The effect of the buffer is based on the conversion of the oxonium ions or hydroxide ions supplied by the acid or base to weak acids or bases, which themselves have little tendency to form $H_3O^+$ or $OH^-$ ions.

The buffer system of the present invention can be any system, contained in the beads, which in contact with a solvent, preferably water, can provide or produce a combination of a weak acid and its conjugate base, or *vice versa.*

A buffer system according to the present invention can for example be a combination of a weak acid and its conjugate base, or *vice versa,* which is contained in the beads. This embodiment is particularly preferred if the effective pH value of the beads should be in a neutral range (pH 6-8).

In a preferred embodiment of the invention the weak acid and/or the weak base are amphoteric compounds, *i.e.* substances, which can react both as an acid and as a base, *e.g.* polyprotic acids such as $NaH_2PO_4$.

In a preferred embodiment of the invention, the buffer system is distributed within the bead. In a method for manufacturing a pharmaceutically active pellet comprising the bead of this preferred embodiment, the pharmaceutically active agent can be deposited on this bead.

In another preferred embodiment of the invention, the buffer system is coated on the surface of the bead. In a method for manufacturing a pharmaceutically active pellet comprising the bead of this preferred embodiment, the pharmaceutically active agent can be deposited on the coated bead.

In a preferred embodiment, the buffer system can comprise a weak acid and the pharmaceutically acceptable carrier material acts as the conjugate base, which is contained in the beads. Accordingly, the pharmaceutically acceptable carrier material can act as a weak acid and the buffer system can comprise the conjugate base.

In a preferred embodiment, the buffer system can comprise a weak base and the pharmaceutically acceptable carrier material acts as the conjugate acid, which is contained in the beads. Accordingly, the pharmaceutically acceptable carrier material can act as a weak base and the buffer system can comprise the conjugate acid.

A weak acid is a proton containing compound, which only partially dissociates when it is dissolved in a solvent such as water. Weak acids have a $pK_a$-value > 8 and weak bases a $pK_b$-value of > 14.

A buffer is nearly exhausted if the ratio of the concentrations of weak acid and conjugate base or *vice versa* exceeds 1:10 to 10:1. Therefore, in the above mentioned embodiment, the inventive beads preferably have concentrations of weak acid and conjugate base or *vice versa* within this range. As the buffer capacity is highest at an equimolar ratio of weak acid and conjugate base or *vice versa,* in a preferred embodiment of the present invention the ratio of concentrations of weak acid and conjugate base or *vice versa* is in a range of 1:5 to 5:1, more preferred in a range of 3:1 to 1:3, even more preferred in a range of 2:1 to 1:2. Most preferred is an equimolar ratio. In this context, concentration means the amount of substance (mol) per weight unit of beads (g). In case any of the above mentioned acids or base has more than one acidic/basic function, the equivalent concentration (normality) has to be used for the calculation of the above ratios.

In another preferred embodiment of the invention, the buffer system of the beads comprises a weak acid. Further, the buffer system contains a strong/weak base, which is not the conjugate base of the weak acid, but which in contact with a solvent, preferably water, can react with the weak acid to its conjugate base in order to form a combination of a weak acid and a conjugate base.

In a preferred embodiment of the invention, the pharmaceutically acceptable carrier material acts as a weak acid, and the buffer system contains a strong/weak base, which is not the conjugate base of the weak acid, but which in contact with a solvent, preferably water, can react with the weak acid to its conjugate base in order to form a combination of a weak acid and a conjugate base.

In a preferred embodiment of the invention, the buffer system contains a weak acid, and the pharmaceutically acceptable carrier material acts as a strong/weak base, which is not the conjugate base of the weak acid, but which in contact with a solvent, preferably water, can react with the weak acid to its conjugate base in order to form a combination of a weak acid and a conjugate base.

In another preferred embodiment of the invention, the buffer system of the beads comprises a weak base. Further, the buffer system contains a strong/weak acid, which is not the conjugate acid of the weak base, but which in contact with a solvent, preferably water, can react with the weak base to its conjugate acid in order to form a combination of a weak base and a conjugate acid.

In another preferred embodiment of the invention, the pharmaceutically acceptable carrier material acts as a weak base. Further, the buffer system contains a strong/weak acid, which is not the conjugate acid of the weak base, but which in contact with a solvent, preferably water, can react with the weak base to its conjugate acid in order to form a combination of a weak base and a conjugate acid.

In another preferred embodiment of the invention, the buffer system contains a weak base. Further, the pharmaceutically acceptable carrier material acts as a strong/weak acid, which is not the conjugate acid of the weak base, but which in contact with a solvent, preferably water, can react with the weak base to its conjugate acid in order to form a combination of a weak base and a conjugate acid.

In another preferred embodiment of the invention, the buffer system of the beads comprises a weak base. Further, the buffer system contains a strong/weak acid, which is not the conjugate acid of the weak base, but which in contact with a solvent, preferably water, can react with the weak base to its conjugate acid in order to form a combination of a weak base and a conjugate acid.

Embodiments with beads comprising a combination of a weak base and a strong/weak acid, which is not the conjugate acid of the weak base, or *vice versa,* are particularly preferred if the effective pH value of the beads should be either acidic or basic (pH $\leq$ 6 or pH $\geq$8).

In a preferred embodiment, the weak acid and strong/weak base or *vice versa* are contained in the beads in an amount that in contact with water, the conjugate base/acid is formed to an extent that the ratio of weak acid/base and conjugated base/acid does not exceed 1:10 to 10:1. As the buffer capacity is highest at an equimolar ratio of weak acid and conjugate base or *vice versa,* in a preferred embodiment of the present invention the ratio is in a range of 1:5 to 5:1, more preferred in a range of 3:1 to 1:3, even more preferred in a range of 2:1 to 1:2. Most preferred is an equimolar ratio. In case any of the above mentioned acids or base has more than one acidic/basic function the equivalent concentration (normality) has to be used for the calculation of the above ratios.

The buffering effect can be quantified by the so-called buffer capacity ($\beta$), which is the moles of strong acid or base necessary to change the pH of 1 litre of a solution by one pH unit. The buffer capacity can be calculated with the following equation

$$\beta = \frac{d(c)}{d(pH)}$$

wherein $\beta$ is the buffer capacity, d(c) is the concentration of acid or base (mol/L), which caused the change of pH and d(pH) is the change of pH values.

For the inventive beads, the buffer capacity can be determined by producing a 10 % w/w solution of beads in 1 litre water and measuring the amount of strong acid/base necessary to conduct a pH change of 1 unit.

In a preferred embodiment of the invention, the buffer capacity of a 10 % w/w suspension of the inventive beads in water is at least $6*10^4$ mol/L, preferably $1*10^{-3}$ mol/L, more preferably $2*10^{-3}$ mol/L and most preferably $4*10^{-3}$ mol/L.

In general, the buffer system is more cost intensive than the pharmaceutically acceptable carrier material, which can for example be a polymeric matrix. Therefore, in a preferred embodiment of the present invention, the amount of buffer in the inventive beads is in the range of 0.1 to 20 wt.-%, more preferred in a range of 0.1. to 10 wt.-%, even more preferred in a range of 0.2 to 7 wt.-% and most preferred in a range of 1 to 5 wt.-%.

Further, buffer systems are regularly water-soluble, which means that a high content of buffer system can lead to a disintegration and/or dissolution of the beads. Therefore, it is preferred that the pharmaceutically acceptable carrier material is insoluble in water and the weight ratio of pharmaceutically acceptable carrier material to buffer system is at least 10:1, preferably at least 20:1, more preferred at least 30:1 and most preferred at least 50:1.

In a preferred embodiment, the beads do not swell or disintegrate, do not change their size or shape significantly when stirred with water for 24 hours. Additionally, moisture uptake, even under conditions of a high relative humidity (above 90% RH), is limited and does not exceed 3.5 - 4 % w/w.

For this, the pharmaceutically acceptable carrier material of the beads has to be insoluble in water and the weight ratio of pharmaceutically acceptable carrier material to buffer system is at least 10:1.

It is particularly preferred if the beads do not disintegrate when being placed in water at a temperature of 25 $\pm$ 1°C and being kept there for 24 h. Particularly, the beads according to this preferred embodiment are characterized in that after having been placed in water at a temperature of 25 $\pm$ 1°C and being kept there for 24 h while being stirred with a speed of 50 rpm $\geq$ 90 wt.-% of the spherical beads still have essentially the same particle size as before.

Particularly, the circle equivalent diameter of the beads reduces by less than 1%. The circle equivalent (CE) diameter is the diameter of a circle with the same area as the particle: CE diameter = $(4A/\pi)^{1/2}$, where A is a particle projected area. The particle projected area is measured with the help of a suitable optical microscope equipped with software allowing image analysis.

Furthermore, the surface roughness remains essentially the same. Particularly, the value of surface roughness of the beads reduces by less than 1%.

Such properties make the beads of the present invention ideal candidates for further coating, especially with water solutions or suspension without the risk of any damage to the surface of the beads or any negative effect on the properties of multiparticulate dosage forms being prepared from the inventive spherical beads.

### Feature c)

To ensure a sufficient buffering effect, the amount of buffer system has to be at least 0.1 wt.-% in relation to the total weight of the beads. In the context of the invention, "total weight of the beads" is the total weight of the dry beads, whereby "dry" means that the content of volatile components (e.g. water), which can be analysed as described above under feature a) and expressed as LoD, is less than 0.1 wt.-%.

The higher the amount of the buffer system in relation to the total weight of the beads, the higher is the buffering effect.

In some applications, the pharmaceutically active pellets comprising the inventive beads are exposed to environments of varying pH in the body, e.g. in the gastrointestinal tract. Therefore, the amount of buffer system is preferably at least 0.2 wt.-%, more preferred at least 1 wt.-%, even more preferred at least 5 wt.-% and most preferred at least 7 wt.-% or even at least 10 wt.-%. In a preferred embodiment of the invention, the pharmaceutically acceptable carrier material can be an acid or base, which is a component of the buffer system. In another preferred embodiment of the invention the pharmaceutically acceptable carrier material is not an acid or base, which is a component of the buffer system. In a preferred embodiment of the invention, the buffer system is at the same time the pharmaceutically acceptable carrier material. In another preferred embodiment of the invention, the buffer system is not the pharmaceutically acceptable carrier material, *i.e.* the beads comprise a pharmaceutically acceptable carrier material and at least one additional component of the buffer system.

### Feature d)

The inventive beads are intended for the deposition of a pharmaceutically active agent on the surface. A pharmaceutically active pellet with a layer of pharmaceutically active agent is more active than a pellet, in which the agent is dispersed. Therefore, the inventive beads should not contain any or at least only low amounts of pharmaceutically active agent.

In a preferred embodiment of the invention, the beads comprise less than 0.2 wt.-% active pharmaceutical ingredients, more preferred less than 0.1 wt.-%, even more preferred less than 0.05 wt.-% and most preferred less than 0.02 wt.-% or even no pharmaceutical ingredients, whereby wt.-% is in in relation to the total weight of the beads.

As used herein, the term "pharmaceutically active agent" refers to any agent, drug, compound, composition or mixture, which provides some pharmacologic effect that can be demonstrated *in-vivo* or *in vitro.* A substance exhibiting a "pharmacologic effect" is capable of interacting with any type of cells present in or on the body of a human or animal, including both, the body's own cells or other cells, such as bacteria, viruses or parasites. Due to its pharmacologic effect, a pharmaceutically active agent is capable of reconstituting, rectifying or positively influencing a physiological function of the body in order to prevent, shorten, partially or completely remedy a disorder, or to alleviate or eliminate the symptoms of the disorder.

The buffer system of the present invention comprises at least one of the compounds selected from the group consisting of acetic acid, acetates, such as sodium acetate, ammonia, ammonium salts such as ammonium chloride, mono-, di- or tribasic phosphates, such as mono-, di- or tribasic sodium- or potassium phosphate, citric acid, phosphoric acid, boric acid, sodium citrate, sodium-, potassium-, magnesium-, calcium bicarbonates, sodium-, potassium-, magnesium-, calcium carbonates, especially $CaCO_3$, tartaric acid, sodium-potassium tartrate, hydroxides, such as sodium-, potassium-, magnesium-, calcium hydroxide and mixtures of the above.

Particularly preferred are the compounds selected from the group consisting of mono-, di- or tribasic phosphates, citric acid, carbonates, sodium-, potassium-, magnesium-, calcium hydroxides.

Particularly prefered are buffer systems consisting of the following components:

1) acetic acid, sodium acetate,
2) ammonia, ammonium chloride,
3) monobasic sodium phosphate, dibasic sodium phosphate, tribasic sodium phosphate
4) monobasic potassium phosphate, dibasic potassium phosphate, tribasic potassium phosphate

5) sodium citrate, citric acid, phosphoric acid, boric acid,

6) sodium carbonate, sodium bicarbonate,

7) tartaric acid, sodium-potassium tartrate.

The buffer beads of the present invention can be produced by adding the weak acid and its conjugate base or a weak base and its conjugate acid during the manufacturing process of the beads.

In a preferred embodiment of the invention, the beads comprise more than one pharmaceutically acceptable carrier material.

In a preferred embodiment of the invention, the beads contain more than one buffer system consisting of a weak acid and its conjugate base or *vice versa.*

By creating different buffer systems, varying the concentrations and ratios of weak acid and conjugate base or vice versa beads with an acidic, neutral or basic pH environment can be obtained. This means that the pH environment of the beads can be adjusted exactly to the need of the pharmaceutically active agent. For example, a combination of sodium carbonate and sodium bicarbonate allows producing highly alkaline beads.

The pH created by the beads according to this invention can be assessed by measuring the pH value of the liquid formed after filtering out solid matter from a 10 % w/w suspension of crushed beads in 1 liter of water. The buffer capacity can be measured by titration with the accurately measured amount of 0.1 M hydrochloric acid or 0.1 M sodium hydroxide to the same liquid and simultaneous determining the pH values. The value of buffer capacity $\beta$ can be calculated in accordance with the above-mentioned equation.

In a preferred embodiment of the invention, the pharmaceutically acceptable carrier material is selected from the following compounds in anhydrous or hydrate form

1) sugars and sugar alcohols such as lactose, dextrin, glucose, sucrose, mannitol, sorbitol, sodium-, potassium-, magnesium-, calcium carbonates, especially $CaCO_3$, as well as other

2) inorganic compounds such as calcium, magnesium silicates, sodium chloride, potassium chloride, as well as other

3) calcium hydrogen phosphate, magnesium hydrogen phosphate and other phosphoric acid salts,

4) microcrystalline cellulose, cellulose and its derivatives

5) starch and its derivatives

6) silicon dioxide, silica compounds

7) stearic acid and its salts

8) natural and synthetic polymers, and mixtures of the above.

Particularly preferred are microcrystalline cellulose (MCC), lactose, starch, carnauba wax, isomalt, inorganic materials such as calcium hydrogen phosphate, aluminium hydroxide, aluminum silicate, calcium and magnesium silicates, sodium chloride, potassium chloride, calcium salts of phosphoric acid, magnesium oxide, magnesium hydroxide, magnesium carbonate (dibasic), sugar compounds such as lactose, dextrin, glucose, sucrose, mannitol, sorbitol, microcrystalline cellulose, cellulose and its derivatives, starch and its derivatives, silicon dioxide and silica compounds, stearic acid and its salts, natural and synthetic polymers or a combination thereof.

In an even more preferred embodiment of the present invention, the pharmaceutically acceptable carrier material is selected from the group consisting of MCC, lactose, starch, calcium phosphates or a combination thereof.

In another preferred embodiment of the invention, the buffer system is enclosed in a solid matrix comprising the pharmaceutically acceptable carrier material.

In a particularly preferred embodiment of the invention, the pharmaceutically acceptable carrier material consists of an organic carrier material and phosphoric acid calcium salt, whereby the beads comprise at least or more than 50 wt.-% of phosphoric acid calcium salt. These embodiments of the present invention stand out due to their high density and/or hardness and their smooth surface and low surface area in comparison to other embodiments of the invention. In specific embodiments of the invention, the carrier material comprises at least or more than 60 wt.-%, of at least or more than 70 wt.-% or of at least or more than 80 wt.-% of phosphoric acid calcium salt. In some embodiments of the invention, the maximum amount of phosphoric acid calcium salt in the carrier material is 79 wt.-%, 89 wt.% or even 99 wt.%.

The phosphoric acid calcium salt can be selected from the group consisting of monobasic calcium phosphate, dibasic calcium phosphate, tribasic calcium phosphate and mixtures thereof. In some embodiments, the phosphoric acid calcium salt is anhydrous or contains water of hydration. Accordingly, the phosphoric acid calcium salt used can be anhydrous, a hydrate or mixtures thereof.

The organic material can be selected from the group consisting of microcrystalline cellulose, lactose, gelatin, cellulose and its derivatives, starch and its derivatives, saccharides and polysaccharides, sugar alcohols (e.g. mannitol, sorbitol), synthetic polymers and mixtures thereof. In some embodiments, the organic material is anhydrous or contains water of hydration. Accordingly, the organic material used can be anhydrous, a hydrate or mixtures thereof.

According to a preferred embodiment of the present invention, the spherical beads have a sphericity of at least or more than 0.80. In more preferred embodiments of the invention, the spherical beads have a sphericity of at least or more than 0.90 or even a sphericity of at least or more than 0.95. The sphericity (circularity) of the spherical beads is determined by an image analysis by measuring a degree of which the particle is similar to a perfect circle. Sphericity (circularity) is calculated from the equation: $4\pi A / P2$, where A is the particle area and P is its perimeter. Particle area and its perimeter are measured with the help of suitable optical microscope equipped with software allowing image analysis.

A high degree of sphericity of the inventive beads allows better homogenous distribution of pharmaceutically active agents on the bead surface. In cases where the pharmaceutically active agent is deposited on the bead surface in the form of a layer, a more even layer thickness can be achieved over the entire bead surface.

In a preferred embodiment of the invention, the pharmaceutically acceptable carrier material of the beads is insoluble in water. In this context, insoluble means that <1 g, preferably < 0.5 g, more preferably < 0.1 g and most preferably < 0.01 g of the carrier material dissolve in 1 liter water at room temperature over a duration of 24 hours. Due to the insolubility in water, the inventive beads of this preferred embodiment do not dissolve upon contact with water. Further, undesirable sticking of the particles, which results in formation of agglomerates is prevented. By using insoluble beads, the coating process can be performed with aqueous solutions or suspensions even at high spray rates without impacting the beads' surface and negatively affecting the properties of multiparticulate dosage forms. In addition, processing time for layering the beads with pharmaceutically active agents can be shortened. Such water insoluble carrier materials are well-known in the art, e.g. calcium phosphates and microcrystalline cellulose, silica compounds and silicon dioxide.

However, in some applications, water-soluble beads are desirable. In these cases, the pharmaceutically acceptable carrier material of the beads has to be water-soluble. Such materials are well-known in the art, e.g. sugar or polyol based compounds.

In a preferred embodiment of the invention, the beads contain less than 0.5 wt.-% sugar, preferably less than 0.2 wt.-%, more preferred less than 0.1 wt.-%, most preferred less than 0.05 wt.-% or even no sugar. In this context, "sugar" means any kind of mono-, di- or polysaccharides. Beads with high amounts of sugar can be problematic for diabetic patients. In addition, especially at elevated temperature, sugars can trigger the Maillard reaction with amine containing pharmaceutically active agents.

In a preferred embodiment of the invention, $\geq$ 90 wt-% of the beads have a particle size in the range of from 90 $\mu$m to 1200 $\mu$m. In specific embodiments of the invention, the particle size is in the range of from 100 $\mu$m to 1100 $\mu$m or in the range of from 100 $\mu$m to 1000 $\mu$m. The particle size of the inventive beads is determined by sieve analysis as indicated in European Pharmacopoeia (Ph. Eur.), 9th Edition.

According to a preferred embodiment of the present invention, the beads have a bulk density of at least or more than 800 g/l. In specific embodiments of the invention, the spherical beads have a bulk density of at least or more than 900 g/l, at least or more than 1000 g/l or even at least or more than 1100 g/l. The bulk density of the inventive beads is determined in a dry graduated 250 ml cylinder according to the procedures as indicated in European Pharmacopoeia (Ph. Eur.), 9th Edition.

Beads with a density enable production of very heavy drug products with prolonged residence time in the stomach. Prolonged gastric residence time is curtail for many pharmaceutically active agents which are locally active in the stomach or have a narrow absorption window in the stomach or in the upper small intestine.

Further, elevated density of the beads accordingly allow a significant reduction of the size/volume of finished dosage forms (MUPS tablets or capsules) which in turn improves a patient compliance and safety of application.

In a preferred embodiment, the inventive beads have a specific surface area of at most or less than 15 m$^2$/g. In specific embodiments of the invention, the specific surface area is at most or less than 10 m$^2$/g, 8 m$^2$/g or 7 m$^2$/g. The specific surface area of the beads is determined by calculating the amount of gas adsorbed on the surface of the beads according to the procedures as indicated in European Pharmacopoeia (Ph. Eur.), 9th Edition.

A low specific surface area of the beads is an indicator for the beads having a very dense and smooth surface. Accordingly, the risk of solutions or suspensions of pharmaceutically active agent to be deposited on the surface of the beads flowing or penetrating into pores on the bead surface is very low. This is important for controlling the amount of pharmaceutically active agent deposited on the surface of the beads as well as its homogenous distribution on the surface, which has an influence on release time and bioavailability of the agent.

The inventive beads are suitable for producing pharmaceutically active pellets being prepared by depositing a pharmaceutically active agent on the surface of said beads. In specific embodiments of the invention, the pharmaceutically active agent is deposited on the surface of the beads by means of depositing a solution, a suspension or a powder of said pharmaceutically active agent on the surface of the beads. In even further specific embodiments, the solution, the suspension or the powder of the pharmaceutically active agent form a layer on the surface of the beads.

In light of the above, any pharmaceutically active pellets in accordance with the present invention consist of the inventive beads having deposited on their surface a pharmaceutically active agent. As said pellets are mainly composed of the inventive beads, they do substantially have the same physical characteristics, such as spherical shape, bulk density, specific surface area, hardness, degree of sphericity, surface roughness, etc., as the inventive beads alone, i.e. without any pharmaceutically active agent, deposited on their surface.

In a preferred embodiment of the invention, the pharmaceutically active agent is deposited on the surface of the beads by means of depositing a solution, a suspension or a powder of said pharmaceutically active agent on the surface of the beads. In a more preferred embodiment, the solution, suspension comprises a binder material, preferably a polymeric binder material.

In another preferred embodiment of the invention, the pharmaceutically active agent is coated on the surface of the beads. Appropriate techniques for coating the surface of the beads are for example Wurster fluid bed coating.

In a preferred embodiment of the invention, the solution, the suspension or the powder of the pharmaceutically active agent forms a layer on the surface of the beads.

The present invention is also directed to a method for producing the inventive beads. The beads are obtained by mixing the carrier material, the buffer system and water in a mixing device for at least 1 minute and up to 10 minutes, thereby producing a mixture. The temperature of said mixture is increased by 4 to 6 °C per minute by means of the mechanical mixing energy applied by the mixing device to the mixture, only.

Increasing the temperature of mixture by means of the mechanical mixing energy applied by the mixing device to the mixture, only, means that there is no external heat input to the mixture by means of e.g. an external heating source being located outside the mixing vessel. Further, there is no heating through the mixing vessel wall. Besides, the mixture is not preheated before being fed into the mixing vessel. The increase in temperature can be obtained by high-speed spinning action.

This high-energy mixing process yields beads with elevated bulk density of at least 800 g/l, preferably at least 1000 g/l and a high degree of sphericity of at least 0.8 and a very smooth surface/low porosity with specific surface area of at most 10 $m^2$/g. In addition, the obtained beads have a high hardness of at least 600 g/$mm^2$.

Pharmaceutically acceptable starting carrier materials used as the carrier material in the inventive method are commonly available on the market. They do not require any special treatment or processing before mixing the carrier material and water in the mixing device.

In a preferred embodiment of the inventive manufacturing process, neither organic solvents nor catalysts are used. So, there are neither residual solvents nor residual catalysts in the finished spherical beads of the invention that can have a detrimental effect on the stability of the pharmaceutically active agent.

After mixing, the wet material can be transferred to a spheronizer and be spheronized to further enhance sphericity of the beads.

After mixing and optional spheronizing, the material is dried to constant weight, preferably in a fluid-bed drier until value of LoD determined at a moisture analyzer balance at 105 °C was below 1 - 2 %.

Optionally, the obtained beads can be sieved after drying.

The inventive method yields beads with a narrow particle size distribution. Accordingly, in a specific embodiment of said method, beads are produced, wherein ≥ 90 wt.-% of the beads have a particle size in the range of from 90 μm to 1200 μm.

The mixing device used for production of spherical beads according to a preferred embodiment of the present invention allows high-speed mixing leading to direct micro-pelletizing. The high-speed mixing action is implemented through a mixing tool, wherein said mixing tool has one or more mixing blades. In specific embodiments of the invention, the rotational speed of the outermost part of the mixing blade is higher than 15 m/s, higher than 20 m/s or even higher than 25 m/s.

In preferred embodiments, the mixing tool is arranged eccentrically in a rotating mixing pan, wherein the mixing pan rotates in the opposite direction as compared to the direction of preferably parallel rotation of the mixing tool, wherein the rotational speed is at least 30 rpm, at least 40 rpm or at least 45 rpm. In particularly preferred embodiments of the invention, the mixing pan is arranged at an angle in the range of from 15 to 45°, most preferred in the range of from 25 to 35.

As compared to the common wet granulation process, the high-energy mixing process according to the present invention releases far more energy by heat, i.e. is highly exothermic. This can be observed by the fast increase of temperature of the mixture during the process. During high-speed mixing, increase in temperature of the mixture reaches 4-6 °C/min. Depending on how long the mixing process is performed, this may result in the mixture reaching temperatures of more than 30 °C, more than 40 °C, more than 50 °C or even more than 60 °C. In specific embodiments of the invention, the maximum temperature reached in the mixing process is less than 70 °C, less than 60 °C, less than 50 °C or even less than 40 °C.

Forces involved in the high-speed mixing process of the invention cause not only rapid temperature increase but also strong compression of powders which apparently results in higher density and lower porosity of the spherical beads obtained. Therefore, with this method, beads with the above mentioned bulk density, specific surface area, and hardness can be obtained. Further, the high-energy mixing partially or completely removes water or other solvents present in the mixture.

Moreover, due to the specific constitution of the beads obtained by this process, their moisture content is very low and they do not tend to water uptake. Therefore, beads of the invention can be suitable for processing water-sensitive active agents. In some embodiments of the invention, the moisture content is less than 2 wt.% or even less than 1 wt.%, wherein the moisture content of the inventive spherical beads is expressed as loss on drying and is determined by thermogravimetric analysis, wherein the spherical beads are dried using a sensitive electrobalance under the temperature of 105 °C to constant mass. The moisture content is defined as the percentage loss in weight of the sample.

Even if the use of a high-energy mixing device is particularly preferred, also other granulations techniques, e.g. high-shear granulators, fluid bed granulators or extrusion or agglomeration in a pelletizer can be used to produce the inventive beads.

When performing the commonly used wet granulation equipment such as high shear granulator or fluid bed granulator the energy involved in the process is however not sufficient to densify a powder mass in the same way as in the high-energy mixing process. This results in a decreased bonding strength between particles. The common wet granulation process produces granules of irregular shape and relatively high porosity. Such granules are agglomerates of insufficient mechanical strength and very broad particle size distribution, from very fine particles to big lumps.

Surprisingly, it was found that the specific mixing process of the invention allows direct preparation of spherical beads of favorable physical characteristic, namely a bulk density of at least 800 g/l, preferably at least 1000 g/l and/or a specific surface area of at most 15 m$^2$/g.

Moreover it allows using commonly available standard raw materials which do not require any special treatment or processing adjusting their functional properties prior further processing.

Beads obtained by the high-energy mixing process have a surface roughness of at least or more than 0.90. In specific embodiments of the invention, the spherical beads have a surface roughness of at least or more than 0.92 or even a surface roughness of at least or more than 0.95. The surface roughness is determined by measurement of particle edge roughness (convexity) and is calculated by dividing the convex hull perimeter by the actual particle perimeter using the equation: Pc / P, where Pc is defined as the convex hull perimeter and P the actual perimeter. P and Pc are measured with the help of suitable optical microscope equipped with software allowing image analysis.

The low surface roughness of the beads obtained by this process allows better homogenous distribution of pharmaceutically active agents on the bead surface. In cases where the pharmaceutically active agent is deposited on the bead surface in the form of a layer, a more even layer thickness can be achieved over the entire bead surface.

The inventive method is simple and very fast, and the beads obtained by the process are spherical enough and can be directly dried, wherein the drying step can performed in different types of dryers including dry dryer, fluid bed dryer, rotary kiln, etc. However, it was observed that the fastest and the most reliable is fluid bed drying process. The temperature of drying should be sufficient to remove the water from the beads. In specific embodiments, the drying temperature is above 60 °C, more preferably above 70°C and most preferably above 80°C. The content of moisture in the final spherical beads is preferably lower than 2 % w/w, and more preferably lower than 1 % w/w.

After the drying step, the pellets can be screened between two sieves, preferably 90 $\mu$m and 1200 $\mu$m, more preferably 100 $\mu$m and 1100 $\mu$m and most preferably 100 $\mu$m and 1000 $\mu$m. According to the present invention, $\geq$ 90 wt.-% of the spherical beads have a particle size in the range of from 90 $\mu$m to 1200 $\mu$m. In specific embodiments of the invention, the particle size is in the range of from 100 $\mu$m to 1100 $\mu$m or in the range of from 100 $\mu$m to 1000 $\mu$m. The particle size of the inventive spherical beads is determined by sieve analysis as indicated in European Pharmacopoeia (Ph. Eur.), 9th Edition, 2.9.38. "Particle-size Distribution Estimation by Analytical Sieving". The spherical beads according to the present invention are free-flowing and are larger than 75 $\mu$m and in this case mechanical sieving is most suitable without necessity of using other means of agitation such as air-jet sieving for example. The material passes readily through sieves so dry sieving method by mechanical agitation can be applied.

Optionally, in order to further improve the degree of sphericity of the beads, a short spheronization step can be performed before drying. However, usually spheronization is not required in order to obtain the desired physical characteristics of the inventive spherical beads. Accordingly, in some embodiments of the invention, the inventive spherical beads having the physical characteristics as indicated above are obtainable without any spheronization step.

In one embodiment of the inventive method, the buffer system can be processed into beads, i.e. the buffer system itself is the pharmaceutically acceptable carrier material.

In another embodiment, the beads comprise the buffer system and an additional pharmaceutically acceptable carrier material, which is not a buffer system. Preferably the buffer system is enclosed in a solid matrix consisting of a pharmaceutically acceptable carrier material.

The invention also concerns the use of a buffer in beads coated with a pharmaceutically active agent.

However, the inventive beads cannot only be used in pharmaceutical, but also non-pharmaceutical formulations, e.g. nutraceutical, nutritional or agricultural formulations. For example, the inventive beads can be coated with a crop protection agent, which necessitates a certain pH environment.

The following examples show some of the features of specific embodiments of the present invention. However, the skilled reader will understand that those embodiments are just exemplary but do not restrict the inventive idea to exactly the combination of features of the embodiments of the examples.

**Examples**

Example 1 - 4 Preparation of acidic buffer beads

Mixtures containing microcrystalline cellulose and either dibasic calcium phosphate or lactose were placed in a mixing pan of a high-energy mixing device (Eirich, intensive mixer type RV01). Then a buffer system consisting of citric acid either alone or with disodium hydrogen was added. Exact compositions are given in Table 1. After addition of water, the powder mass was blended for around 10 minutes. A mixing pan was rotated clockwise with the speed of 45 rpm. A mixing tool was rotated counterclockwise with the speed of 27 m/s. The amount of water used depends on the ratio between components. Wet material was transferred to a spheronizer and spheronized for 10 minutes with a speed of 1500 rpm. Obtained micro pellets were dried in a fluid-bed drier at a temperature of 80 °C for around 20 minutes - until value of loss on drying determined at a moisture analyzer balance at 105 °C was below 1 - 2 wt.-%. Dry micro pellets were classified between sieves of 100 $\mu$m and 1000 $\mu$m.

**Table 1.** Examples of acidic buffer beads

|  | Example 1 | Example 2 | Example 3 | Example 4 |
|---|---|---|---|---|
| Dibasic calcium phosphate anhydrous | 63.0 % | 64.0 % | 77.0 % | - |
| Microcrystalline cellulose | 30.0 % | 30.0 % | 20.0 % | 20.0 % |
| Lactose monohydrate | - | - | - | 77.0 % |
| Citric acid monohydrate | 7.0 % | 5.15 % | 2.57 % | 2.57 % |
| Disodium hydrogen phosphate | - | 0.85 % | 0.43 % | 0.43 % |
| Effective pH (10 % suspension) | 3.6 | 3.5 | 4.3 | 2.8 |
| Buffer capacity (10 % w/w suspension vs. 0.1 M NaOH) [mol/L] | $7.1 \cdot 10^{-3}$ | $4.7 \cdot 10^{-3}$ | $1.0 \cdot 10^{-3}$ | $1.1 \cdot 10^{-3}$ |
| *Bulk density (g/L)* | *> 1000* | *> 1000* | *> 1000* | *> 800* |

Example 5 - 8 Preparation of neutral buffer beads

Mixtures containing microcrystalline cellulose and either dibasic calcium phosphate or lactose were placed in a mixing pan of a high-energy mixing device (Eirich, intensive mixer type RV01). Then a buffer system consisting of disodium hydrogen phosphate and either citric acid or monosodium hydrogen phosphate was added. Exact compositions are given in Table 2. After addition of water, the powder mass was blended for around 10 minutes. A mixing pan was rotated clockwise with the speed of 45 rpm. A mixing tool was rotated counterclockwise with the speed of 27 m/s. The amount of water used depends on the ratio between components. Wet material was transferred to a spheronizer and spheronized for 10 minutes with a speed of 1500 rpm. Obtained micro pellets were dried in a fluid-bed drier at a temperature of 80 °C for around 20 minutes - until value of loss on drying determined at a moisture analyzer balance at 105 °C was below 1 - 2 %. Dry micro pellets were classified between sieves of 100 $\mu$m and 1000 $\mu$m.

Advantage of functional buffer pellets according to this innovation (Examples 5-7) can be illustrated by comparing the buffer capacity with commercial inert starter pellets consisting only of dibasic calcium phosphate anhydrous and microcrystalline cellulose (Example 8). Buffer capacity values are significantly higher (more than 10 times) for the buffer pellets. Inert cores possess very limited buffer capacity compared to the buffer beads.

**Table 2.** Examples of beads of neutral buffer beads

| | Example 5 | Example 6 | Example 7 | Example 8 |
|---|---|---|---|---|
| Dibasic calcium phosphate anhydrous | 64.0 % | - | 77.0 % | 80.0 % |
| Microcrystalline cellulose | 30.0 % | 30.0 % | 30.0 % | 20.0 % |
| Lactose monohydrate | - | 67.0 % | - | - |
| Citric acid monohydrate | 0.60 % | - | - | - |
| Disodium hydrogen phosphate | 5.40 % | 1.5 % | 1.5 | - |
| Monosodium dihydrogen phosphate | - | 1.5 % | 1.5 | - |
| Effective pH (10 % suspension) | 7.3 | 7.5 | 7.4 | 7.3 |
| Buffer capacity (10 % w/w suspension vs. 0.1M HCl) [mol/L] | $4.2 \cdot 10^{-3}$ | $1.1 \cdot 10^{-3}$ | $2.2 \cdot 10^{-3}$ | $2.8 \cdot 10^{-4}$ |
| Buffer capacity (10 % w/w suspension vs. 0.1M NaOH) [mol/L] | $1.8 \cdot 10^{-3}$ | $1.0 \cdot 10^{-3}$ | $1.1 \cdot 10^{-3}$ | $1.5 \cdot 10^{-4}$ |
| Bulk density (g/L) | > 1000 | > 800 | > 1000 | > 1000 |

**Claims**

1. **Beads** for use in producing pharmaceutically active pellets being prepared by depositing a pharmaceutically active agent on the surface of said beads, wherein the beads comprise

   a) a pharmaceutically acceptable carrier material and
   b) a buffer system,
   wherein
   c) the amount of the buffer system is at least 0.1 wt.-% in relation to the total weight of the beads and
   d) the beads comprise less than 0.5 wt.-% active pharmaceutical agent in relation to the total weight of the beads.

2. **Beads** according to claim 1, wherein the buffer system contains at least one component selected from the group consisting of acetic acid, acetates, such as sodium acetate, ammonia, ammonium salts such as ammonium chloride, mono-, di- or tribasic phosphates, such as mono-, di- or tribasic sodium or potassium phosphate, citric acid, phosphoric acid, boric acid, sodium citrate, sodium carbonate, sodium bicarbonate, tartaric acid, sodium-potassium tartrate, hydroxides, such as hydroxides, such as sodium-, potassium-, magnesium-, calcium hydroxide and mixtures of the above.

3. **Beads** according to any one of the preceeding claims, wherein the pharmaceutically acceptable carrier material is selected from the group consisting of carnauba wax, isomalt, inorganic materials such as calcium hydrogen phosphate, aluminum hydroxide, aluminum silicate, calcium and magnesium silicates, sodium chloride, potassium chloride, calcium salts of phosphoric acid, magnesium oxide, sodium-, potassium, magnesium-, calcium bicarbonates, sodium-, potassium-, magnesium-, calcium carbonates, especially $CaCO_3$, sugars such as lactose, dextrin, glucose, sucrose, mannitol, sorbitol, microcrystalline cellulose, cellulose and its derivatives, starch and its derivatives, silicon dioxide and silica compounds, stearic acid and its salts, natural and synthetic polymers or a combination of the above.

4. **Beads** according to any one of the preceeding claims, wherein the beads are spherical, preferably with a sphericity of at least 0.8.

5. **Beads** according to any one of the preceeding claims, wherein the pharmaceutically acceptable carrier material is insoluble in water.

6. **Beads** according to any one of the preceeding claims, wherein $\geq$ 90 wt.-% of the beads have a particle size in the range of from 90 $\mu$m to 1200 $\mu$m.

7. **Beads** according to any one of the preceeding claims with a bulk density of at least 800 g/l, preferably > 1000 g/l.

8. **Beads** according to any one of the preceeding claims with a specific surface area of at most 10 m$^2$/g.

9. **Pharmaceutically active pellets** being prepared by depositing a pharmaceutically active agent on the surface of the beads according to any one of claims 1 to 8.

10. **Pharmaceutically active pellets** according to claim 9, **characterized in that** the pharmaceutically active agent is deposited on the surface of the beads by means of depositing a solution, a suspension or a powder of said pharmaceutically active agent on the surface of the beads.

11. **Pharmaceutically active pellets** according to claim 10, **characterized in that** the solution, the suspension or the powder of the pharmaceutically active agent forms a layer on the surface of the beads.

12. **Method** for producing beads according to any of claims 1 to 8, wherein the spherical beads are obtained by mixing the carrier material, the buffer system and water in a mixing device for at least 1 minute and up to 10 minutes, thereby producing a mixture, wherein the temperature of said mixture is increased by 4 to 6 °C per minute by means of the mechanical mixing energy applied by the mixing device to the mixture, only.

13. **Use** of a buffer system in beads, which are coated with a pharmaceutically active agent.

Europäisches
Patentamt

European
Patent Office

Office européen
des brevets

# EUROPEAN SEARCH REPORT

Application Number

EP 20 16 8193

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | EP 2 223 702 A1 (TOMITA PHARMA [JP]) 1 September 2010 (2010-09-01) * the whole document * * claims 1-17 * | 1-11,13 | INV. A61K9/16 A61K9/50 |
| X,D | EP 2 659 882 A1 (TOMITA PHARMA [JP]) 6 November 2013 (2013-11-06) * the whole document * * claims 1-11 * | 1-11,13 | |
| X | WO 2010/059506 A1 (MALLINCKRODT BAKER INC [US]; DEORKAR NANDU [US] ET AL.) 27 May 2010 (2010-05-27) * the whole document * * claims 1-24; examples 1-9 * | 1-11,13 | |
| X | WO 2004/032901 A1 (VECTURA LTD [GB]; TOBYN MICHAEL [GB] ET AL.) 22 April 2004 (2004-04-22) * the whole document * * claims 1-146 * | 1-11,13 | |
| A | BOWEN P: "Particle Size Distribution Measurement from Millimeters to Nanometers and from Rods to Platelets", JOURNAL OF DISPERSION SCIENCE AND TECHNOLOGY, TAYLOR AND FRANCIS GROUP, NEW YORK, NY, US, vol. 23, no. 5, 1 January 2002 (2002-01-01), pages 631-662, XP009102859, ISSN: 0193-2691, DOI: 10.1081/DIS-120015368 * the whole document * | 1-13 | TECHNICAL FIELDS SEARCHED (IPC) A61K |

The present search report has been drawn up for all claims

| Place of search | Date of completion of the search | Examiner |
|---|---|---|
| Munich | 27 August 2020 | Felder, Christian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 16 8193

This annex lists the patent family members relating to the patent documents cited in the above-mentioned European search report.
The members are as contained in the European Patent Office EDP file on
The European Patent Office is in no way liable for these particulars which are merely given for the purpose of information.

27-08-2020

| Patent document cited in search report | | | Publication date | Patent family member(s) | | | Publication date |
|---|---|---|---|---|---|---|---|
| EP 2223702 | A1 | 01-09-2010 | EP | 2223702 | A1 | 01-09-2010 | |
| | | | JP | 5757492 | B2 | 29-07-2015 | |
| | | | JP | 2014196361 | A | 16-10-2014 | |
| | | | JP | WO2009072334 | A1 | 21-04-2011 | |
| | | | US | 2010260856 | A1 | 14-10-2010 | |
| | | | WO | 2009072334 | A1 | 11-06-2009 | |
| EP 2659882 | A1 | 06-11-2013 | CN | 103313703 | A | 18-09-2013 | |
| | | | EP | 2659882 | A1 | 06-11-2013 | |
| | | | JP | 5578534 | B2 | 27-08-2014 | |
| | | | JP | 5910949 | B2 | 27-04-2016 | |
| | | | JP | 2014169318 | A | 18-09-2014 | |
| | | | JP | WO2012091040 | A1 | 05-06-2014 | |
| | | | US | 2014030346 | A1 | 30-01-2014 | |
| | | | WO | 2012091040 | A1 | 05-07-2012 | |
| WO 2010059506 | A1 | 27-05-2010 | AU | 2009316876 | A1 | 07-07-2011 | |
| | | | BR | PI0921078 | A2 | 15-12-2015 | |
| | | | CA | 2744377 | A1 | 27-05-2010 | |
| | | | CN | 102215823 | A | 12-10-2011 | |
| | | | EP | 2365799 | A1 | 21-09-2011 | |
| | | | JP | 2012509326 | A | 19-04-2012 | |
| | | | JP | 2014148556 | A | 21-08-2014 | |
| | | | KR | 20110086741 | A | 29-07-2011 | |
| | | | SG | 171362 | A1 | 28-07-2011 | |
| | | | TW | 201023894 | A | 01-07-2010 | |
| | | | US | 2011229527 | A1 | 22-09-2011 | |
| | | | WO | 2010059506 | A1 | 27-05-2010 | |
| WO 2004032901 | A1 | 22-04-2004 | AU | 2003269284 | A1 | 04-05-2004 | |
| | | | CA | 2500923 | A1 | 22-04-2004 | |
| | | | EP | 1549294 | A1 | 06-07-2005 | |
| | | | JP | 2006506357 | A | 23-02-2006 | |
| | | | KR | 20050089961 | A | 09-09-2005 | |
| | | | NZ | 539277 | A | 30-04-2008 | |
| | | | US | 2006127480 | A1 | 15-06-2006 | |
| | | | WO | 2004032901 | A1 | 22-04-2004 | |
| | | | ZA | 200502693 | B | 24-10-2005 | |

EPO FORM P0459

For more details about this annex : see Official Journal of the European Patent Office, No. 12/82

**EP 3 892 262 A1**

**Patent documents cited in the description**

- EP 2223702 A1
- EP 2659882 A1
- EP 2496261 A

**Non-patent literature cited in the description**

- European Pharmacopoeia (Ph. Eur.)
- Particle-size Distribution Estimation by Analytical Sieving. European Pharmacopoeia (Ph. Eur.)